# EUROPEAN PATENT SPECIFICATION

(11) **EP 3 530 289 B1**
(45) Date of publication and mention of the grant of the patent: **29.03.2023**
(21) Application number: 19386009.5
(22) Date of filing: 25.02.2019
(51) Int. Cl.: A61K 47/10, A61K 47/26, A61K 9/08, A61K 31/00

(54) **ORAL PHARMACEUTICAL SOLUTIONS COMPRISING MELATONIN**
ORALE PHARMAZEUTISCHE LÖSUNGEN MIT MELATONIN
SOLUTIONS PHARMACEUTIQUES ORALES COMPRENANT DE LA MÉLATONINE

(30) Priority: 26.02.2018 GR 20180100084
(43) Date of publication of application: 28.08.2019
(73) Proprietor: Lamda Laboratories S.A., 19400 Koropi Attikis (Karellas) (GR)
(72) Inventor: KARATZAS, Aggelos, Vrilissia, GR15235 (GR); STAPPA, Argyro, Chalandri, GR15234 (GR); RESVANI, Amalia, Ano Petralona, GR11852 (GR)

(56) References cited:
- WO-A1-2015/144965
- WO-A1-2016/058985
- WO-A2-2013/068565
- DATABASE GNPD [Online] MINTEL; 23 July 2007 (2007-07-23), anonymous: "Liquid Sleeping Aid Drops", XP055600005, retrieved from www.gnpd.com Database accession no. 743321
- DATABASE GNPD [Online] MINTEL; 24 June 2019 (2019-06-24), anonymous: "Melatonin + Chamomile & Lavender Dietary Supplement with Wildberry and Vanilla Flavor", XP055600004, retrieved from www.gnpd.com Database accession no. 6648559

## Description

### TECHNICAL FIELD

The present invention relates to stable pharmaceutical solutions suitable for oral administration comprising melatonin.

### BACKGROUND OF THE INVENTION

Melatonin or N-acetyl-5-methoxytryptamine (Chemical formula I), is chemically named N-[2-(5-Methoxy-1H-indol-3-yl)ethyl]acetamide.

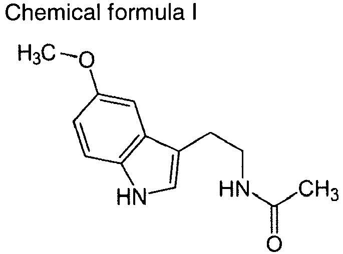

Melatonin is a naturally occurring hormone produced by the pineal gland and is structurally related to serotonin. The hormone secretion increases soon after the onset of darkness, peaks between 2 and 4 a.m., and gradually falls during the second half of the night. Along with multiple neurotransmitters (e.g. serotonin, dopamine, norepinephrine), it plays an important role in the regulation of circadian rhythms that are responsible for the sleep-wake cycle. Abnormalities in the balance of said chemical messenger are frequently experienced by individuals, disrupting the normal sleep pattern. Jet lag, shift work syndrome, seasonal affective diseases and sleep disturbances due to neurological, neurodevelopmental, psychiatric and behavioural disorders are amongst the typical causes for the circadian rhythm disorders. For the amelioration of the relevant clinical manifestations, the administration of exogenous synthetic melatonin has been proven as a safe and effective pharmacological option, efficiently restoring the reduced levels of the nocturnal melatonin.

Melatonin is only slightly soluble in water, with an aqueous solubility value of 2 mg/ml at 20°C (Scientific Committee on Consumer Safety. Opinion on melatonin, March 2010) while it is soluble in ethyl alcohol, DMSO and dimethylformamide. WO 86/05093 discloses a 1 mg/ml solution of melatonin in 30% ethyl alcohol for the topical treatment of skin diseases. Use of this solvent in oral adult medicines is discouraged for a number of health reasons including interactions with other medicines, diseases, effect on driving performance, issues with addiction, pregnancy and breast feeding (EMA/CHMP/507988/2013, Committee for Human Medicinal Products, 23 January 2014).

Lee et al (Lee B-J et al. Arch. Pharm. Res. 1997, Vol.20, No.6, pages 560 to 565) have studied the solubility of melatonin in propylene glycol. According to the authors, the solubility of melatonin was enhanced in a mixture of propylene glycol at 25 degrees Celsius (°C), increasing slowly as a function of propylene glycol concentration, until reaching 40% propylene glycol and then steeply increased. In addition, it was shown that melatonin exhibits pH-independent solubility in water.

Another long-appreciated problem with aqueous solutions of melatonin is that of instability during storage over a period of time (Lee. B-J et al. Arch. Pharm. Res. 1997, Vol.20, No.6, pages 560 to 565; Daya, S. et al. J. Pineal Res. 2001, Vol. 31, pages 155 to 158). U.S. Pat. No. 5,939,084 describes oral solutions in a mixture of butylene glycol (20.00%) and Propylene glycol (15.00%) which were stable for at least four weeks at 4 °C. Considering the high costs associated with storing and distributing refrigerated medications, it is highly desired to produce liquid formulations that are stable at room temperature and even exhibit enhanced stability at higher temperatures.

WO 2015/144965 A1 discloses a pharmaceutically acceptable liquid composition comprising propylene glycol, polyethylene glycol and melatonin or a derivative, a salt, a prodrug, or a solvate thereof.

WO 2013/068565 A2 discloses a pharmaceutical composition in the form of a solution characterised in that it is obtained by dissolving a powder comprising melatonin in an amount from 35 to 90% in weight, at least one water soluble excipient in an amount from 5 to 60% in weight and at least one water soluble surfactant in an amount from 0.5 to 5% of the total weight of the powder, in a mixture of water and propylene glycol, in which melatonin is present in an amount from 3 to 30 mg/ml and the polyalkylene glycol is present in an amount from 5 to 40% of the total volume of the liquid used.

WO 2016/058985 A1 discloses a composition characterized in that a diluted solution at the sought concentration of melatonin is achieved by admixing a precise volume of melatonin at 100 mg/1.0 ml with an appropriate volume of the diluting fluid purified water for pharmaceutical use to yield a medicinal or nutraceutical composition for enteral use whenever a therapeutic effective dose of melatonin is required by oral administration.

Technically an even bigger challenge is the development of effectively preserved formulations which at the same time do not contain large quantities of antimicrobial preservatives which inevitably raise safety and toxicity issues in patients. This is particularly relevant for pharmaceutical preparations that address chronic health problems such as sleep disorders. A review of over-the-counter melatonin products available in the market in the form of a drinking solution shows that this requirement is yet to be achieved. A document retrieved from the MINTEL GNPD Database discloses an oral pharmaceutical solution *(Liquid Sleeping Aid Drops* marketed by Tridea Products) comprising melatonin, propylene glycol, sorbitol and purified water, wherein the pH of the oral solution is sour. The composition of said solution comprises glycerin, benzoates and sorbate. A separate document retrieved from the MINTEL GNPD Database discloses an oral pharmaceutical solution (*ZzzQuil Pure Zzzs* marketed by Procter & Gamble) comprising melatonin, propylene glycol, sorbitol and purified water, wherein the pH of the oral solution is sour. The compositions disclosed in this separate document comprise sodium benzoate.

Moreover, melatonin is a molecule prone to air oxidation. Thus, a significant issue arises when oral solutions prone to atmospheric oxidation are provided in multi-dose containers because of the risk of microbial contamination, or physicochemical degradation of the solution, once the closure system has been breached, due to repeated opening and closing after first use by the patient. It is very difficult to formulate oxygen-sensitive drugs such as melatonin in multi-dose vials, which are sealed with a polymer stopper. Polymers are reasonably permeable to oxygen. Thus, oxidatively unstable drugs require more than just oxygen exclusion during sealing to prevent oxidation from occurring.

The present invention addresses the problems of prior art knowledge by providing aqueous oral pharmaceutical solutions of melatonin which exhibit excellent shelf life while at the same time achieving sufficient antimicrobial activity using minimal excipients.

### SUMMARY OF THE INVENTION

The present invention provides stable aqueous pharmaceutical solutions suitable for oral administration comprising melatonin in association with a pharmaceutically acceptable liquid carrier.

According to the invention, the oral pharmaceutical solutions comprise melatonin as active ingredient, propylene glycol within the range from 20 mg/ml to 500 mg/ml, sorbitol within the range from 20 mg/ml to 400 mg/ml and purified water, wherein the pH of the oral solution is from 3.0 to 5.0 and wherein the oral solution is free of parabens, glycerol, sorbic acid, sodium or potassium benzoate.

The use of propylene glycol at specific concentration ranges with simultaneous pH adjustment in the 3.0 to 5.0 range results in physicochemically stable aqueous oral solutions of melatonin while at the same time providing sufficient antimicrobial activity.

The aqueous oral pharmaceutical solutions of melatonin according to the invention have the advantage of being physicochemically stable while providing sufficient antimicrobial activity without using ethanol, polyethylene glycol, sodium or potassium benzoate, benzoic acid, sorbic acid as well as methyl p-hydroxybenzoate, propyl-p-hydroxybenzoate, ethyl-p-hydroxybenzoate and the sodium salts of these esters.

### DETAILED DESCRIPTION OF THE INVENTION

The present invention provides stable aqueous pharmaceutical solutions suitable for oral administration comprising melatonin in association with a pharmaceutically acceptable liquid carrier.

As used throughout the present description and claims, the term «mg/ml», when referring to the active ingredient or inactive ingredients (excipients), means the milligrams of active or inactive ingredient per ml of oral solution.

As used throughout the present description and claims, the concentrations of sorbitol in the aqueous solutions of melatonin correspond to the concentration of anhydrous sorbitol.

Shelf life after first use (In-use shelf life), as established by the document of European Medicines Agency "Note for Guidance on the in-use stability testing of human medicinal products" (CPMPQWP/2934/99), is a period of time during which a multi-dose product can be used whilst retaining quality within an accepted specification once the container is first opened.

The ability of antimicrobial preservatives to inhibit the growth of, or kill, microorganisms in pharmaceutical preparations is evaluated with Antimicrobial Efficacy Tests (AETs). As used throughout the present description, the term "AET" refers to the test for efficacy of antimicrobial preservation described in paragraph 5.1.3 of the European Pharmacopoeia (EP). The test consists of challenging the preparation, with a prescribed inoculum of suitable microorganisms, storing the inoculated preparation at ambient temperature, avoiding sunlight, withdrawing samples from the container at specified intervals of time and counting the microorganisms in the samples so removed. The preservative properties of the preparation are adequate if, under the conditions of the test, there is a significant fall or no increase, as appropriate, in the number of microorganisms in the inoculated preparation after 2, 7, 14 and 28 days. The EP criteria for evaluation of antimicrobial activity in oral preparations are given in the following table in terms of decrease in the number of viable microorganisms relatively to the value obtained for the inoculum.

**Table 1: EP Criteria for AET (limits in log reduction units)**

| | 2 days | 7 days | 14 days | 28 days |
|---|---|---|---|---|
| Bacteria | - | - | 3 | No increase |
| Moulds & Yeasts | - | - | 1 | No increase |

During pre-formulation studies, the inventors studied the solubility of melatonin in aqueous solutions comprising a number of pharmaceutically acceptable solvents at concentrations well within the safety limits for Acceptable Daily Intake released by the WHO. Propylene glycol was identified as an important component of the liquid carrier and its level was studied and optimized in the course of the development.

Extensive testing was conducted during formulation studies to identify excipients that, when combined with propylene glycol, achieve optimal thermal stability under extreme and accelerated conditions, i.e. 55°C & 40°C/ 75%RH for a period of 15 and 30 days, respectively. The generated data disclosed herein show that the addition of sorbitol enhances the stabilizing action of propylene glycol, leading to melatonin aqueous compositions with very low impurity levels even under thermal stress.

The pH of the liquid carrier was identified as a critical parameter for the maintenance of the chemical and physical integrity of the product. At pH range between 3.0 and 5.0 the liquid carrier exhibits optimal thermal stability as evidenced by the very low impurity levels.

Importantly, the compositions of the present invention do not comprise additional excipients such as ethanol, parabens, sodium bisulfite and sodium formaldehyde sulfoxylate or other stabilizing excipients, which may raise additional safety and toxicity issues.

According to the invention, the oral pharmaceutical solutions comprise melatonin as active ingredient, 20 mg/ml to 500 mg/ml propylene glycol and 20 mg/ml to 400 mg/ml sorbitol, wherein the pH of the oral solution is from 3.0 to 5.0 and wherein the oral solution is free of parabens, glycerol, sorbic acid, sodium or potassium benzoate.

The concentration of propylene glycol is preferably within the range from 100 mg/ml to 400 mg/ml.

The concentration of sorbitol is preferably within the range from 100 mg/ml to 300 mg/ml.

The oral pharmaceutical solutions according to the invention comprise melatonin at a concentration from 0.1 mg/ml to 10.0 mg/ml. The concentration of melatonin in the compositions of the present invention is preferably from 1 mg/ml to 6 mg/ml.

Sorbitol may be added in the oral pharmaceutical solutions according to the invention in the form of sorbitol crystalline powder or crystallizing sorbitol liquid or partially dehydrated crystallizing sorbitol liquid or non-crystallizing sorbitol liquid as described in the corresponding Ph. Eur. Monographs. Preferably, sorbitol is non-crystallizing sorbitol liquid (i.e. sorbitol aqueous solution 70% w/w) as described in the corresponding Ph. Eur. monograph.

The compositions of the present invention exhibit excellent stability even though they do not contain additional stabilizing agents. Thus, the aqueous pharmaceutical solutions of melatonin of the present invention do not contain additional stabilizing agents, beyond propylene glycol and sorbitol, that may raise additional safety and toxicity issues.

It was surprisingly found that the vehicle itself possesses antimicrobial properties, rendering the addition of preservatives unnecessary. It is important that such compositions do not include additional excipients such as ethanol, polyethylene glycol, sodium benzoate, benzoic acid, sorbic acid as well as methyl p-hydroxybenzoate, propyl p-hydroxybenzoate, ethyl-p-hydroxybenzoate and the sodium salts of these esters, which may raise additional safety and toxicity issues.

The oral aqueous pharmaceutical solutions of melatonin, according to the invention may also optionally contain additional excipients commonly used in preparing oral liquid compositions, such as viscosity adjusting agents, natural sweetening agents, non-sugar based artificial sweetening agents and flavouring agents.

The viscosity adjusting agents may be, for example, sodium carboxymethylcellulose, methylcellulose, hydroxypropylcellulose, polyvinylpyrrolidone, maltitol or any combination thereof.

The natural sweetening agents that may be used in the present invention comprise, amongst others, erythritol, xylitol, mannitol, maltitol, fructose, glucose, sucrose, maltose, or any combination thereof.

The non-sugar based artificial sweetening agents which may be used in the present invention comprise, amongst others, sucralose, saccharin sodium, saccharin, aspartame, acesulfame potassium or any combination thereof.

Appropriate flavouring agents which may be used in the present invention comprise, amongst others, any of the many non-toxic natural or artificial flavouring agents known in the daily practice. In particular, the flavouring agents used may include one or more of a variety of natural or artificial fruit flavours. Alternatively, or in addition, the flavouring agents may include one or more of natural or artificial vanilla, chocolate, and caramel flavourings, amongst others.

Surprisingly, the use of antioxidants, such as sodium metabisulfite and ethylenediamine tetraacetic acid ("EDTA"), was found not to be desirable, because their incorporation in the formulation resulted in extensive decomposition of melatonin. This behavior may be associated with the increased reactivity of the said components giving rise to active species that actually exacerbate the oxidation reaction. Thus, antioxidants and chelating agents were excluded from the compositions of the present invention.

Melatonin oral aqueous pharmaceutical solutions of the present invention exhibit excellent in-use shelf life (i.e. shelf life after first use) when supplied in multi-dose containers. Specifically, they remain physicochemically stable and at the same time effectively preserved when stored at room temperature (20°C - 25°C) even when the containers are opened at least once a day for at least two months.

The compositions of the present invention may be prepared using methods well-known in the prior art. For example they may be prepared using the following process: Half of the specified amount of purified water is added to the main vessel. The amount of propylene glycol is charged under continuous stirring. The specified quantity of melatonin is transferred to the above solution and mixed until complete dissolution. The quantities of sucralose, sorbitol and strawberry flavour are added sequentially. The pH is checked and, if required, adjusted to 3.0 - 5.0 with sodium hydroxide 0.1N. The volume of the solution is adjusted to the desired batch volume by adding purified water. The final solution is mixed and filtered through a 35 - 40 µm cellulose membrane or polypropylene cartridge filter. The solution is filled into the designated containers, preferably under nitrogen purging.

### EXAMPLES

The following examples show the influence of the proposed carrier, according to the invention, on the stability of melatonin and on the preservation of the oral solutions.

### EXAMPLE 1

Melatonin exhibits low and pH-independent solubility in water, thus the development of a liquid carrier with sufficient solubilisation capacity for the label claim of interest constituted prerequisite for product development. To efficiently address the formulation challenge, the solubilizing potential of a series of pharmaceutically acceptable solvents was investigated. The attributes of assay and appearance were selected as metrics for assessing the dissolution process of melatonin (1 mg/ml) in every single vehicle. The study and the results are tabulated below. The mixing times required for the delivery of a clear solution of melatonin were also recorded.

**Table 2 Solubility study results**

| **Trial ID** | | **Appearance** | **Assay (%)** | **Mixing time (min)** |
|---|---|---|---|---|
| Trial A | No solvent | Undissolved particles in solution | 90.5 | NA* |
| Trial B | Propylene glycol 50 mg/ml | Undissolved particles in solution | 94.1 | NA |
| Trial C | Propylene glycol 100 mg/ml | Clear colourless solution | 98.4 | 30 |
| Trial D | Propylene glycol 200 mg/ml | Clear colourless solution | 98.9 | 30 |
| Trial E | Propylene glycol 400 mg/ml | Clear colourless solution | 98.7 | 20 |
| Trial F | Glycerol 400 mg/ml | Undissolved particles in solution | 93.0 | NA |
| Trial G | Povidone K30 10 mg/ml | Undissolved particles in solution | 83.1 | NA |
| Trial H | Kolliphor RH 40 15 mg/ml | Clear colourless solution | 98.2 | 120 |
| Trial I | Sorbitol 400 mg/ml | Undissolved particles in solution | 69.3 | NA |

| | | | | |
|---|---|---|---|---|
| *After 90 minutes mixing, insoluble matter was visible. | | | | |

According to the experimental results of Table 2, concentrations of propylene glycol above 100 mg/ml were found adequate for the production of a clear melatonin solution when propylene glycol was the only organic solvent used. Similarly, complete dissolution of melatonin was observed when the surfactant Kolliphor RH 40 was used, however prolonged mixing times were recorded. The remaining excipients of glycerol and sorbitol were judged sub-optimal for the purpose. Overall, propylene glycol was treated as an integral component of the liquid carrier and its level was further studied and optimized in the course of the development.

### EXAMPLE 2

The impact of the pH parameter on the stability performance of melatonin oral solution was the objective of this study. For the identification of the most favourable pH region, a set of trials buffered at different pH values were prepared and then incubated at stressed and accelerated conditions. Attributes such as appearance and related substances % were selected as meaningful stability indicators. The concentration of related substances was estimated by HPLC. The relevant results are tabulated in Table 3.

**Table 3 Determination of pH on stability performance**

| **Component** | **Compositions (mg/ml)** | | | | | |
|---|---|---|---|---|---|---|
| | **1** | **2** | **3** | **4** | **5** | **6** |
| Melatonin | 1.0 | 1.0 | 1.0 | 1.0 | 1.0 | 1.0 |
| Propylene Glycol | 100.0 | 100.0 | 400.0 | 100.0 | 100.0 | 100.0 |
| HCl or NaOH sol 0.1 N | QS to pH 3.0 | QS to pH 4.0 | QS to pH 5.0 | QS to pH 6.0 | QS to pH 7.0 | QS to pH 8.0 |
| Purified Water | Qs to 1 ml | | | | | |

| **Related Substances (%) T=0** | | | | | | |
|---|---|---|---|---|---|---|
| 5-methoxytryptamine | ND | ND | ND | ND | ND | ND |
| Max unknown impurities | 0.01 (RRT:0.96) | 0.02 (RRT:1.32) | 0.03 (RRT:2.30) | 0.03 (RRT:1.13) | 0.02 (RRT:1.13) | 0.07 (RRT:1.22) |
| Total | 0.03 | 0.07 | 0.06 | 0.11 | 0.09 | 0.18 |

| **Related Substances (%) 30 days, 40°C ± 2°C/75% RH ± 5% RH** | | | | | | |
|---|---|---|---|---|---|---|
| 5-methoxytryptamine | 0.03 | 0.01 | 0.01 | 0.02 | 0.03 | 0.05 |
| Max unknown impurities | 0.03 (RRT:1.44) | 0.08 (RRT:0.83) | 0.06 (RRT:0.83) | 0.29 (RRT:0.56) | 0.34 (RRT:0.56) | 0.39 (RRT:0.56) |
| Total | 0.18 | 0.24 | 0.19 | 0.82 | 1.27 | 1.42 |

| | | | | | | |
|---|---|---|---|---|---|---|
| ND: Not detected; RRT: Relative retention time | | | | | | |

Provided that gradual increase of unknown impurities was evidenced while increasing the pH value of the preparation, it was deduced that for the maintenance of the chemical and physical integrity of the product the acidic environment was imperative. Thus, and in line with the above results, the pH area within the range from 3.0 to 5.0 was initially selected as the optimal.

### EXAMPLE 3

The stability of melatonin in aqueous solution has been studied (Lee B-J et al. Arch. Pharm. Res. 1997, Vol.20, No.6, pages 560 to 565; Daya S. et al. J. Pineal Res. 2001, Vol. 31, pages 155 to 158), classifying it as a rather sensitive molecule. For this reason an extensive compatibility study comprising a large variety of excipients was conducted. Each composition was placed into stability chambers under extreme and accelerated conditions, i.e. 55°C & 40°C/ 75%RH for a period of 15 and 30 days, respectively, and the levels of the related substances produced were used as the responses of the study.

**Table 4 Compatibility study protocol**

| **Component** | **Compatibility Protocol (mg/ml)** | | | | | |
|---|---|---|---|---|---|---|
| | **1** | **2** | **3** | **4** | **5** | **6** |
| **Melatonin** | 1.0 | 1.0 | 1.0 | 1.0 | 1.0 | 1.0 |
| **Propylene Glycol** | - | 100.0 | 100.0 | 100.0 | 100.0 | 100.0 |
| **Sorbitol 70%** | - | - | 400.0 | - | - | - |
| **Liquid Maltitol** | - | - | - | 400.0 | - | - |
| **Sodium metabisulfite** | - | - | - | - | 1.0 | - |
| **Disodium EDTA** | - | - | - | - | - | 1.0 |
| **HCl or NaOH sol 0.1 N** | QS to pH 5.0 | | | | | |

| **Related Substances (%) T=0** | | | | | | |
|---|---|---|---|---|---|---|
| **5- methoxytryptamine** | ND | BRL | BRL | BRL | ND | BRL |
| **Max unknown impurities** | 0.01 (RRT:1.22) | 0.03 (RRT:2.30) | 0.01 (RRT:2.57) | 0.03 (RRT:1.06) | 2.54 (RRT:0.51) | 0.01 (RRT:0.51) |
| **Total** | 0.06 | 0.04 | 0.04 | 0.12 | 2.96 | 0.02 |

| **Related Substances (%) T=30 days, 40°C ± 2°C/75% RH ±5% RH** | | | | | | |
|---|---|---|---|---|---|---|
| **5-methoxy-tryptamine** | 0.02 | 0.01 | 0.01 | 0.01 | 0.14 | 0.01 |
| **Max unknown impurities** | 0.45 (RRT:0.56) | 0.06 (RRT:0.83) | 0.05 (RRT:0.83) | 0.31 (RRT:0.56) | 13.05 (RRT:0.49) | 0.17 (RRT:0.56) |
| **Total** | 1.09 | 0.19 | 0.15 | 0.82 | 15.9 | 0.49 |

Based on the above data, the largest stabilizing effect was exhibited by the solvents of propylene glycol (100.0 mg/ml) and sorbitol (400.0 mg/ml). Other co-solvents such as maltitol and glycerol at the concentrations tested did not effectively synergize with propylene glycol to yield a stabilizing carrier.

On the contrary, the incorporation of antioxidants and chelating agent resulted in extensive decomposition of melatonin; a behavior that can be associated with the increased reactivity of said components giving rise to active species that actually exacerbate the oxidation reaction. Of the tested sweeteners, sucralose was identified comparatively more inert than sodium saccharin while sodium benzoate was found as the most suitable representative from the functional group of preservatives.

### EXAMPLE 4

This example demonstrates the effects of different concentrations of propylene glycol and sorbitol, in a pH within the range from 3.0 to 5.0, on the stability and preservation of the solutions.

The solutions were prepared through a manufacturing process, where half of the specified amount of purified water was added to the main vessel. The amount of propylene glycol was charged under continuous stirring. The specified quantity of melatonin was transferred to the above solution and mixed until complete dissolution. Varying quantities of sorbitol were then added. The pH was checked and, if required, adjusted to the pH shown in Table 4 with sodium hydroxide 0.1 N. The volume of the solution was adjusted to the desired batch volume by adding purified water. The final solution was mixed and filtered through a 35 - 40 µm cellulose membrane or polypropylene cartridge filter. The solution was filled into the designated containers, preferably under nitrogen purging.

The produced compositions were placed into stability chambers under accelerated conditions, i.e. 40°C/ 75%RH for a period of 30 days and 3 months, respectively, and the levels of the related substances produced were used as the responses of the study. The compositions and the stability results obtained are shown in Table 5.

**Table 5 Compositions with varying concentrations of propylene glycol and sorbitol**

| **Composition** | **1** | **2** | **3** | **4** |
|---|---|---|---|---|
| **Propylene Glycol** | 250.0 | 250.0 | 100.0 | 250.0 |
| **Sorbitol** | 100.0 | 241.4 | 200.0 | 100.0 |
| **pH** | 3.5 | 3.5 | 4.0 | 4.2 |

| **Related substances, T = 0** | | | | |
|---|---|---|---|---|
| Max unknown (%) | 0.03 (RRT:1.47) | 0.03 (RRT:1.47) | 0.03 (RRT:1.47) | 0.02 (RRT:1.27) |
| Total Impurities (%) | 0.10 | 0.15 | 0.08 | 0.11 |

| **Related substances, T=30 Days, 40°C ± 2°C/75% RH ± 5% RH** | | | | |
|---|---|---|---|---|
| Max unknown (%) | 0.14 (RRT:0.70) | 0.13 (RRT=0.70) | 0.07 (RRT=0.70) | 0.22 (RRT=0.70) |
| Total Impurities (%) | 0.17 | 0.16 | 0.12 | 0.20 |

| **Related Substances, T=3 Months, 40°C ± 2°C/75% RH ± 5% RH** | | | | |
|---|---|---|---|---|
| Max unknown (%) | 0.05 (RRT:0.57) | 0.15 (RRT:0.57) | 0.13 (RRT:0.57) | 0.16 (RRT:0.57) |
| | 0.12 (RRT:1.35) | 0.10 (RRT:0.70) | 0.10 (RRT:0.70) | |
| Total Impurities (%) | 0.23 | 0.25 | 0.23 | 0.29 |

| | | | | |
|---|---|---|---|---|
| RRT= Relative retention time | | | | |

It is revealed that a combination of propylene glycol and sorbitol, at certain concentration and pH ranges, is effective in preserving and at the same time physicochemically stabilizing aqueous melatonin solutions.

### EXAMPLE 5

Based on the compatibility study results and the optimum pH range for the solution, sodium benzoate was identified as a suitable preservation agent capable of mediating antimicrobial protection in the pH region of interest. Hence, and in accordance with the requirements of the European Pharmacopoeia, its adequacy was determined by means of AETs.

A series of trials were subjected to this challenge testing to identify the minimum quantity needed to deliver a product that maintains its microbiological integrity during its shelf life. For all trials, the compositions contain 150.0 mg/ml propylene glycol, 200 mg/ml sorbitol and 0.2 mg/ml sucralose at pH 3.5 to 4.5. The percentages of sodium benzoate investigated were: 0.5 mg/ml (composition P1), 0.3 mg/ml (composition P2) and 0.0 mg/ml (composition P3). Compositions P1 and P2 do not fall within the scope of the claims of the present invention.

By applying the pour plate method, as described in the relevant chapter of European Pharmacopoeia, the following results (i.e. colonies) were recorded at the time intervals specified in Table 6.

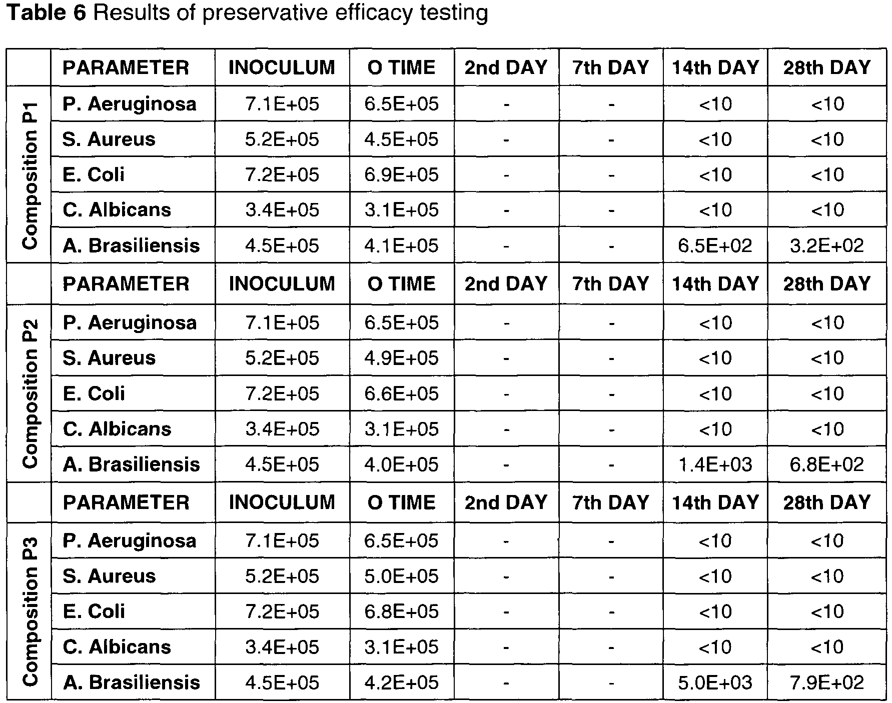

The inventors have surprisingly found that the vehicle itself possesses antimicrobial properties, rendering the addition of sodium benzoate unnecessary. It is apparent that the inclusion of propylene glycol has a significant contribution to the overall preservation; thereby a complementary preservative efficacy testing was executed for generating solid evidence that the optimum for stability amount of propylene glycol 150 mg/ml is also sufficient for the microbial integrity of the preparation.

### EXAMPLE 6

Table 7 shows preferred compositions according to the present invention.

The following solutions were prepared through a manufacturing process, where half of the specified amount of purified water was added to the main vessel. The amount of propylene glycol was charged under continuous stirring. The specified quantity of melatonin was transferred to the above solution and mixed until complete dissolution. The quantities of sucralose, sorbitol and strawberry flavour were added sequentially. The pH was checked and, if required, adjusted to 3.5 - 4.5 with sodium hydroxide 0.1N. The volume of the solution was adjusted to the desired batch volume by adding purified water. The final solution was mixed and filtered through a 35 - 40 µm cellulose membrane or polypropylene cartridge filter. The solution was filled into the designated containers preferably under nitrogen purging.

**Table 7 Preferred compositions according to the present invention**

| | **Composition E1** | **Composition E2** |
|---|---|---|
| **Active ingredient** | mg/ml | |
| Melatonin | 1 | 1 |

| **Excipients** | mg/ml | |
|---|---|---|
| Propylene glycol | 150.0 | 100.0 |
| Sorbitol solution 70% (Non Crystallising) | 200.0 | 200.0 |
| Sucralose | 0.2 | 0.2 |
| Strawberry flavour | 0.5 | 0.5 |
| Hydrochloric acid, 0.1 N solution | QS to pH 3.5 - 4.5 | as to pH 3.5 - 4.5 |
| Purified water | as to 1.0 ml | QS to 1.0 ml |
| Nitrogen | Traces | Traces |

The compositions of Table 7 were studied for two months with regard to its physicochemical stability and preservation effectiveness after first use in storage conditions of 20 - 25°C. The test simulates the use of the drug product in practice since the bottles are uncovered twice a day for two months and a certain quantity of solution is removed each time. Quantification of melatonin and its impurities in the solutions was performed by HPLC. Table 8 shows the stability and AET data obtained for compositions E1 and E2.

**Table 8: Stability after first use (in-use stability) for compositions E1 and E2**

| | **Composition E1** | **Composition E2** |
|---|---|---|
| **Assay (T= 0)** | 102.6% | 101.3% |
| **Assay (T= 2 months)** | 102.4% | 98.2% |
| **5-methoxytryptamine (T= 0)** | B.R.L. | B.R.L. |
| **5-methoxytryptamine (T= 2 months)** | B.R.L. | 0.013 |
| **Total impurities (T= 0)** | B.R.L. | 0.21% |
| **Total impurities (T= 2 months)** | 0.1% | 0.36% |
| **AET (T = 2 months)** | Pass | Pass |

| | | |
|---|---|---|
| B.R.L.: Below reporting limit | | |

The results show excellent in-use shelf life. Specifically, the composition remains physicochemically stable and at the same time effectively preserved when stored at room temperature (20°C - 25°C), even when the containers are opened twice a day for two months.

## Claims

1. Oral pharmaceutical solution comprising melatonin, propylene glycol within the range from 20 mg/ml to 500 mg/ml, sorbitol within the range from 20 mg/ml to 400 mg/ml and purified water, wherein the pH of the oral solution is from 3.0 to 5.0 and wherein the oral solution is free of parabens, glycerol, sorbic acid, sodium or potassium benzoate.

2. Oral pharmaceutical solution according to claim 1, wherein the concentration of propylene glycol is within the range from 100 mg/ml to 400 mg/ml.

3. Oral pharmaceutical solution according to claim 1, wherein the concentration of sorbitol is within the range from 100 mg/ml to 300 mg/ml.

4. Oral pharmaceutical solution according to claim 1 or 2, wherein the concentration of melatonin is from 0.1 mg/ml to 10 mg/ml.

5. Oral pharmaceutical solution according to claim 1 or 2, wherein the concentration of melatonin is from 1 mg/ml to 6 mg/ml.

6. Oral pharmaceutical solution according to any one of claims 1 to 5, wherein sorbitol is non-crystallizing liquid sorbitol.

7. Oral pharmaceutical solution according to any one of claims 1 to 6, which is free of additional stabilizing agents, beyond propylene glycol and sorbitol.

8. Oral pharmaceutical solution according to any one of claims 1 to 7, which consists of
1.0 mg/ml melatonin,
150.0 mg/ml propylene glycol,
200.0 mg/ml sorbitol,
0.2 mg/ml sucralose,
0.5 mg/ml strawberry flavour
and purified water, wherein the pH of the oral solution is from 3.5 to 4.5.

## Patentansprüche

1. Orale pharmazeutische Lösung mit Melatonin, Propylenglykol im Bereich von 20 mg/ml bis 500 mg/ml, Sorbitol im Bereich von 20 mg/ml bis 400 mg/ml und gereinigtes Wasser, wobei der pH-Wert der oralen Lösung 3,0 bis 5,0 beträgt und wobei die orale Lösung frei von Parabenen, Glycerin, Sorbinsäure, Natrium- oder Kaliumbenzoat ist.

2. Orale pharmazeutische Lösung gemäß Anspruch 1, wobei die Konzentration von Propylenglycol im Bereich von 100 mg/ml bis 400 mg/ml liegt.

3. Orale pharmazeutische Lösung gemäß Anspruch 1, wobei die Konzentration von Sorbitol im Bereich von 100 mg/ml bis 300 mg/ml liegt.

4. Orale pharmazeutische Lösung gemäß Anspruch 1 oder 2, wobei die Konzentration von Melatonin zwischen 0,1 mg/ml und 10 mg/ml liegt.

5. Orale pharmazeutische Lösung gemäß Anspruch 1 oder 2, wobei die Konzentration von Melatonin zwischen 1 mg/ml und 6 mg/ml liegt.

6. Orale pharmazeutische Lösung gemäß einem der Ansprüche 1 bis 5, wobei Sorbitol nicht kristallisierendes flüssiges Sorbitol ist.

7. Orale pharmazeutische Lösung gemäß einem der Ansprüche 1 bis 6, die außer Propylenglykol und Sorbitol frei von zusätzlichen Stabilisatoren ist.

8. Orale pharmazeutische Lösung gemäß einem der Ansprüche 1 bis 7, die Folgendes enthält:
1,0 mg/ml Melatonin,
150,0 mg/ml Propylenglycol,
200,0 mg/ml Sorbitol,
0,2 mg/ml Sucralose,
0,5 mg/ml Erdbeeraroma
und gereinigtes Wasser, wobei der pH-Wert der oralen Lösung zwischen 3,5 und 4,5 liegt.

## Revendications

1. Solution pharmaceutique buvable contenant de la mélatonine, du propylène glycol dans la plage de 20 mg/ml à 500 mg/ml, du sorbitol dans la plage de 20 mg/ml à 400 mg/ml et de l'eau purifiée, dont le pH varie de 3,0 à 5,0 et qui est exempte de parabènes, de glycérol, d'acide sorbique, de benzoate de sodium ou de potassium.

2. Solution pharmaceutique buvable selon la revendication 1, dans laquelle la concentration de propylène glycol se situe dans la plage de 100 mg/ml à 400 mg/ml.

3. Solution pharmaceutique buvable selon la revendication 1, dans laquelle la concentration de sorbitol se situe dans la plage de 100 mg/ml à 300 mg/ml.

4. Solution pharmaceutique buvable selon la revendication 1 ou 2, dans laquelle la concentration de mélatonine se situe entre 0,1 mg/ml et 10 mg/ml.

5. Solution pharmaceutique buvable selon la revendication 1 ou 2, dans laquelle la concentration de mélatonine se situe entre 1 mg/ml et 6 mg/ml.

6. Solution pharmaceutique buvable selon l'une des revendications 1 à 5, dans laquelle le sorbitol est du sorbitol liquide non cristallisant.

7. Solution pharmaceutique buvable selon l'une des revendications 1 à 6, exempte d'agents stabilisants additionnels hormis le propylène glycol et le sorbitol.

8. Solution pharmaceutique buvable selon l'une des revendications 1 à 7, composée de
1,0 mg/ml de mélatonine,
150,0 mg/ml de propylène glycol,
200,0 mg/ml de sorbitol,
0,2 mg/ml de sucralose,
0,5 mg/ml d'arôme fraise
et de l'eau purifiée, le pH de la solution buvable étant de 3,5 à 4,5.
